# EUROPEAN PATENT APPLICATION

(11) **EP 1 172 439 A1**
(43) Date of publication of application: **16.01.2002**
(21) Application number: 99912082.7
(22) Date of filing: 31.03.1999
(51) Int. Cl.: C12N 15/54, C12N 9/10, C07K 14/195

(54) **THERMOSTABLE ENZYME HAVING AMINOTRANSFERASE ACTIVITY AND GENE ENCODING THE SAME**

(71) Applicant: National Institute of Advanced Industrial Science and Technology, Tokyo 100-8921 (JP)
(72) Inventor: MATUI, Ikuo, Tsukuba-shi Ibaraki 305-0031 (JP); ISHIKAWA, Kazuhiko, Higashiosaka-shi, Osaka 577-0802 (JP); ISHIDA, Hiroyasu, Ryugasaki-shi Ibaraki 301-0042 (JP); KOSUGI, Yoshitsugu, Tsukuba-shi Ibaraki 305-0061 (JP); MATSUI, Eriko, Tsukuba-shi Ibaraki 305-0031 (JP)
(74) Representative: Woods, Geoffrey Corlett
(86) International application number: JP9901696
(87) International publication number: WO0060096

(57) **Abstract**

An aminotransferase which remains stable at high temperatures and over a wide pH range. Thus, a novel catalyst for an aminotransferase reaction to be carried out under severe conditions is provided.

## Description

### FIELD OF THE INVENTION

The present invention relates to thermostable aminotransferase useful in synthesizing an amino acid derivative with high optical purity, and a gene encoding the enzyme.

### BACKGROUND OF THE PRESENT INVENTION

Aminotransferases are enzymes useful in synthesizing amino acids, amines, and prochiral ketones with high optical purity.

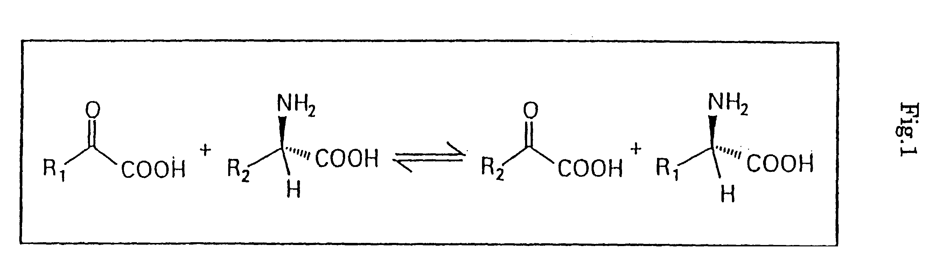

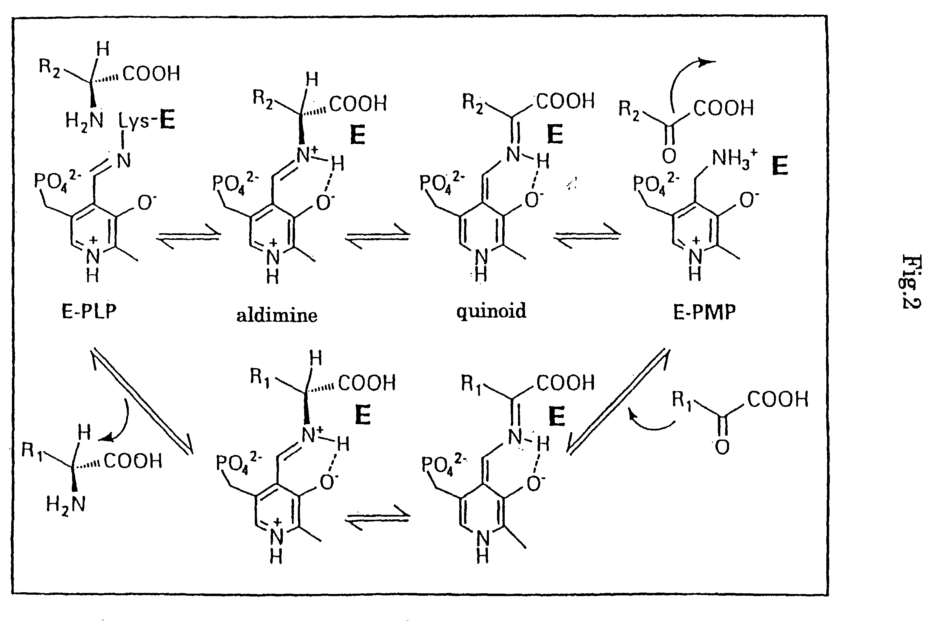

Aminotransferases catalyzes a reaction to produce other oxo acids and amino acids by transferring amino groups of amino acids to α-keto acids (Fig. 1). This reaction synthesizes amino acid derivatives retaining stereoisomerism of amino group donors (Fig. 2).

A variety of aminotransferases with different substrate specificities have been isolated from mammalian cells and yeast cells. However, these transferases have poor heat resistance, acid-resistance and alkali-resistance since most of them are derived from mesophilic organisms. Because of such poor resistance, these aminotransferases were not able to be used for chemical synthesis (e.g. amino acid derivative synthesis) under severe conditions in which organic solvents and the like are used.

Therefore, isolation of aminotransferase which remains stable at high temperature and over a wide pH range can provide very useful, novel catalyst in chemical synthesis (e.g. amino acid derivative synthesis) under severe conditions.

Consequently, development of aminotransferase which remains stable under severe conditions has been desired.

### SUMMARY OF THE INVENTION

The purpose of the present invention is to provide aminotransferase which remains stable at high temperature and over a wide pH range, and a gene encoding the same.

As a result of thorough studies to address the above problems, the present inventors have determined a nucleotide sequence of a chromosomal DNA of an extreme thermophilic bacterium capable of growing at 90°C to 100°C. Then based on the nucleotide sequence the present inventors have isolated a gene predicted to encode a protein having aminotransferase activity. Finally the present inventors have completed the invention by integrating the gene into *E.coli* for expression, and by confirming that the protein encoded by the gene has aminotransferase activity and remains stable at high temperature (90°C or more) and over a wide pH range (pH 4 to pH 11).

Thus, the present invention is an enzyme which:
(1) has aminotransferase activity,
(2) exhibits higher aminotransferase activity when an aromatic amino acid is used as an amino group donor rather than when a non-aromatic amino acid is used as an amino group donor, and
(3) has an optimum temperature of 90°C.

Further, the present invention is an enzyme which:
(1) has aminotransferase activity,
(2) exhibits higher aminotransferase activity when an aromatic amino acid is used as an amino group donor than when a non-aromatic amino acid is used as an amino group donor,
(3) has an optimum temperature of 90°C,
(4) has an optimum PH of 6.0,
(5) maintains its activity even when subjected to treatment at pH 6.5 and 95°C for 6 hours,
(6) has a half-life at pH 6.5 and 110°C of 30 minutes,
(7) remains stable at pH 4 to pH 11 and 25°C for 24 hours or more,
(8) has a melting temperature at pH 6.5 of 120.1°C where molar enthalpy change is 2.4x10³KJ/mole,
(9) has an α-helix content of 40% at pH 6.5 and 25°C,
(10) has molecular weight of 44,000Da,
(11) has a homodimeric subunit structure,
(12) has an isoelectric point of 5.2, and for which;
(13) denaturation is irreversible.

Furthermore, the present invention is a protein which is the following protein (a) or (b):
(a) a protein which comprises an amino acid sequence of SEQ ID NO: 1,
(b) a protein which comprises an amino acid sequence derived from the amino acid sequence of SEQ ID NO: 1 by deletion, replacement or addition of one or more amino acids and has aminotransferase activity.

Moreover, the present invention is a gene which encodes the protein above.

Now the present invention will be described in more detail.

### 1. A first invention is an enzyme which:

(1) has aminotransferase activity,
(2) exhibits higher aminotransferase activity when an aromatic amino acid is used as an amino group donor than when a non-aromatic amino acid is used as an amino group donor,
(3) has an optimum temperature of 90°C,
(4) has an optimum PH of 6.0,
(5) maintains its activity even when subjected to treatment at pH 6.5 and 95°C for 6 hours,
(6) has a half-life at pH 6.5 and 110°C of 30 minutes,
(7) remains stable at pH 4 to pH 11 and 25°C for 24 hours or more,
(8) has a melting temperature at pH 6.5 of 120.1°C where molar enthalpy change is 2.4x10³KJ/mole,
(9) has an α-helix content of 40% at pH 6.5 and 25°C,
(10) has molecular weight of 44,000Da,
(11) has a homodimeric subunit structure,
(12) has an isoelectric point of 5.2, and for which;
(13) denaturation is irreversible.

The enzyme of this invention can be obtained, for example by the following methods.

The cells of a microorganism capable of producing the enzyme of this invention are disrupted, suspended in a buffer, and then centrifuged. The supernatant obtained by the centrifugation is purified by a variety of chromatography based on the presence of aminotransferase activity as an index. Thus, the enzyme of this invention can be obtained.

A buffer and conditions for centrifugation and chromatography employed in the above methods maybe appropriately selected from a normal range employed upon purification of enzymes from microbial cells. The presence of aminotransferase activity can be determined, for example by tracing an increase in absorbance at 412nm resulting from reduction of 5,5' - Dithiobis (2-nitrobenzoic acid) (DTNB) with L-cysteic acid and 2-ketoglutaric acid as substrates.

Any microorganism can be employed in the above methods. That is, microorganisms employed herein are not specifically limited as long as they can produce the enzyme of this invention.

For example, an extreme thermophilic bacterium can be used. Particularly, a sulfur-metabolizing thermophilic archaebacterium, *Pyrococcus horikoshi* (deposited at JAPAN Collection of Microorganism, RIKEN, Accession No.: JCM9974) can be used. In addition, a microorganism (e.g. *E.coli*) into which the gene of this invention has been transferred as described below can be used.

The enzyme of this invention has aminotransferase activity, and remains stable at high temperature and over a wide pH range so that it can be used as a catalyst for aminotransferase reaction under severe conditions.

With very high amino transferase activity for an aromatic amino acid, the enzyme of this invention is particularly useful as a catalyst for aminotransferase reaction using an aromatic amino acid as a substrate.

Aminotransferase reaction using the enzyme of this invention can provide an amino acid derivative with high optical purity.

### 2. A second invention is the following protein (a) or (b):

(a) a protein which comprises an amino acid sequence of SEQ ID NO: 1,
(b) a protein which comprises an amino acid sequence derived from the amino acid sequence of SEQ ID NO: 1 by deletion, replacement or addition of one or more amino acids and has aminotransferase activity.

Here, the number of amino acids represented by "one or more amino acids" is not specifically limited as long as they are deleted, replaced or added by techniques standard at the time when the present application is filed and do not lose aminotransferase activity. Further, a protein in which one or more amino acids are deleted, replaced or added can be produced by techniques standard at the time when the present application is filed, e.g. site-directed mutagenesis (Zoller et al., Nucleic acids Res. 10, 6487-6500, 1982).

A protein of this invention can be obtained by the same steps as employed for the enzyme of this invention,

As with the enzyme of this invention, the protein of this invention has aminotransferase activity in addition to stability at high temperature and over a wide pH range. Hence, the protein of this invention can be used as a catalyst for aminotransferase reaction under severe conditions.

Further, like the enzyme of the present invention, the protein of the present invention has very high aminotransferase activity for an aromatic amino acid, and is particularly useful as a catalyst for aminotransferase reaction using an aromatic amino acid as a substrate.

Like the enzyme of this invention, aminotransferase reaction using the protein of this invention can provide an amino acid derivative with high optical purity.

### 3. A third invention is a gene encoding the protein of the above invention.

For example, the gene of this invention can be obtained as described below.

First, chromosomal DNA is extracted from microorganisms having the gene of this invention. Microorganisms used herein are not specifically limited as long as they have the gene of this invention. Examples of such a microorganism include extreme thermophilic bacteria. More specifically, a sulfur-metabolizing thermophilic archaebacterium, *Pyrococcus horikoshi* (deposited at JAPAN Collection of Microorganism, RIKEN, Accession No: JCM9974) can be used. In addition, chromosomal DNA can be extracted from microorganisms by standard techniques.

Next, the extracted chromosomal DNA is partially digested with restriction enzymes and then inserted into a vector. Restriction enzymes used herein are not specifically limited as long as they can cleave chromosomal DNA to appropriate lengths, such as a length of approximately 40kb. Examples of such restriction enzymes include, but are not limited to, HindIII, EcoRI, SalI, and KpnI. A preferable restriction enzyme is HindIII. A vector used herein is not specifically limited as long as it can function as a cloning vector. Examples of such vectors include pBAC108L and pFOS1.

Subsequently, the above recombinant vector is introduced into an appropriate host cell to construct a genome DNA library, followed by determination' of the nucleotide sequence of chromosomal DNA. Examples of the host cell which can be used herein include, but are not limited to, *Escherichia coli* and yeast cells. A method for introducing a recombinant vector into a host cell may be appropriately selected depending on the vector to be used. For example, electroporation is preferred when pBAC108L is used as a vector; λ phage or the like is preferred when pFOS1 is used as a vector. Further, the nucleotide sequence of chromosomal DNA can be determined by for example, Maxam-Gilbert chemical modification method, dideoxynucleotide chain termination, or modified methods therefrom which are automated.

Then, homologous regions of the protein of this invention are found from the obtained sequence data, and astructural gene encoding the protein of this invention is identified.

Next, primers complementary to both ends of the structural gene above are synthesized and used for PCR to amplify the structural gene so that the gene of this invention can be obtained.

Moreover, the gene of this invention can be chemically synthesized by a known method such as the phosphite triester method.

*Escherichia coli* BL21 PET11a/ArATph into which the gene of this invention is transferred was internationally deposited as FERM BP-6685 at the National Institute of Advanced Industrial Science and Technology (AIST) (1-1-3, Higashi, Tsukuba, Ibaraki, JAPAN) under the Budapest Treaty (deposition date: January 26, 1998).

The gene of this invention encodes the protein of this invention. That is, the gene of this invention is integrated into an expression vector, and the vector is introduced into and expressed in a host cell derived from a prokaryotic or eukaryotic organism, thereby producing the protein of this invention in large quantity. Examples of the expression vectors which can be used herein include pETlla and pET15b. Examples of a host cell derived from prokaryotic organism include *E.coli* (e.g. *E.coli* BL21 (DE3), *E.coli* XL1-BlueMRF' and the like) and *Bacillus Subtilis.* Examples of a host cell derived from an eukaryotic organism which can be used herein include a vertebrate cell and a yeast cell.

### BRIEF DESCRIPTION OF THE DRAWINGS

Figure 1 shows an aminotransferase reaction catalyzed by aminotransferase.
Figure 2 shows the mechanism of an aminotransferase reaction catalyzed by aminotransferase.
Figure 3 shows the relation between temperature and Kapp.

### BEST MODE FOR CARRYING OUT THE INVENTION

Now the present invention will be described more specifically by use of examples, but this invention is not limited by these examples.

### Example 1 Culturing of Bacteria

A sulfur-metabolizing thermophilic archaebacterium, *Pyrococcus horikoshi* (deposited at. JAPAN Collection of Microorganism, RIKEN, Accession No: JCM9974) was cultured as 'described below.

13.5g of salt, 4g of Na₂SO₄, 0.7g of KCl, 0.2g of NaHCO₃, 0.1g of KBr, 30mg of H₃BO₃, 10g of MgCl₂ · 6H₂O, 1.5g of CaCl₂, 25mg of SrCl₂, 1.0ml of resazurin solution (0.2g/l), 1.0g of yeast extract and 5g of bactopeptone were dissolved in 11 of water. Then the solution was adjusted to be pH6.8 and then sterilized under pressure.

Next, dry and heat -sterilized elemental sulfur was added to the solution up to 0.2%. This medium was made anaerobic by saturating with argon, and then JCM9974 was inoculated to the medium. To confirm that the medium became anaerobic, Na₂S solution was added to the medium to see that no pink coloring of resazurin solution resulted from Na₂S in the liquid medium.

Then, JCM9974 was cultured in the above liquid medium at 95°C for 2 to 4 days.

### Example 2 Preparation of Chromosomal DNA

The chromosomal DNA of JCM9974 was prepared by the following methods.

After culturing of JCM9974, cells were collected by centrifugation at 5,000rpm for 10 min. The cells were washed twice with 10mM Tris(pH 7.5) - 1mM EDTA solution, and then sealed into InCert Agarose (FMC) block. The block was treated in a solution containing 1% N-lauroyl sarcosine and 1mg/ml protease K so that chromosomal DNA was separated and prepared in the agarose block.

### Example 3 Construction of Library Clone Containing Chromosomal DNA

The chromosomal DNA obtained in Example 2 was partially digested with restriction enzyme HindIII, and fragments with a length of approximately 40 kb were prepared by means of agarose gel electrophoresis.

Using T4 ligase, the DNA fragments were ligated with Bac vectorpBAC108L (Stratagene) and pFOS1 (Stratagene) both of which had been completely digested with restriction enzymes HindIII.

When the former vector pBAC108L was used, the ligated DNA was immediately introduced into *E.coli* by electroporation.

When the latter vector pFOS1 was used, the ligated DNA was packaged by GIGA Pack Gold (Stratagene) into λ phage particles in a test tube. Then, *E.coli* was infected with the particles, thereby introducing DNA into *E.coli.*

Antibiotic, chloramphenicol-resistant *E.coli* populations obtained by these methods were designated as BAC and Fosmid library, respectively. Clones appropriate for covering chromosomal DNA of JCM9974 were selected from these libraries and clone alignment was performed.

### Example 4 Sequencing of BAC or Fosmid Clone

DNA was recovered from each of the aligned BAC and Fosmid clones. The recovered DNA was fragmented by ultrasonication. The fragmented DNA was subjected to agarose gel electrophoresis, and 1kb and 2kb-long DNA fragments were recovered. These DNA fragments were inserted into HincII restriction enzyme sites of pUC118 plasmid vectors so that 500 shot gun clones were produced per BAC or Fosmid clone.

Nucleotide sequences of each shot gun clone were determined using Perkin Elmer 373 or 377 (manufactured by ABI, automatic device for reading nucleotide sequences). The nucleotide sequences obtained from each shot gun clone were combined and edited using Sequencher (software for automatically combining nucleotide sequences). Therefore, the whole nucleotide sequences of each BAC or Fosmid clone were determined.

### Example 5 Identification of aromatic amino acid aminotransferase Gene

The nucleotide sequences of each BAC or Fosmid clone determined in Example 4 were analyzed by a large-scale computer. Thus a gene (SEQ ID NO: 2) encoding aromatic amino acid, aminotransferase was identified.

### Example 6 Construction of Expression Plasmid

To construct restriction enzyme sites (Ndel and BamHI) before and after a structural gene region, 2 types of DNA primers as shown below were synthesized. PCR was performed using these primers to introduce restriction enzyme sites before and after the structural gene.
Upper primer :
5' - TTTTGTCGACTTACATATGGCGCTAAGTGACAGA - 3'
Lower primer :
5' - TTTTGGTACCTTTGGATCCTTAACCAAGGATTTAAACTAG - 3'

The fragments amplified by PCR were completely digested with restriction-enzymes (Ndel and BamHI) at 37°C for 2 hours, thereby isolating structural genes and the genes were purified.

pETlla (Novagen) was cleaved with restriction enzymes Ndel and BamHI and then purified. Then, the products were allowed to react in the presence of the above structural gene and T4 ligase at 16°C for 2 hours to be ligated. Next, part of the ligated DNA was introduced into competent cells of *E.coli* XL 1-BlueMRF', thereby obtaining colonies of transformants. Expression plasmids were isolated from the obtained colonies, and then purified by the alkaline method.

### Example 7 Expression of Recombinant Genes

The competent cells of *E.coli* (*E.coli* BL21 (DE3), Novagen) were thawed and 0.1ml of the thawed cells was transferred into a Falcon tube. 0.005ml of an expression plasmid solution was added to the cells. The mixture was allowed to stand on ice for 30 min, and then subjected to heat shock at 42°C for 30 sec. 0.9ml of SOC medium was added to the mixture, followed by shaking culture at 37°C for 1 hour. An appropriate quantity of the culture product was inoculated over a 2YT agar plate containing ampicillin and cultured overnight at 37°C, thereby obtaining transformants.

The transformants were cultured in a 2YT medium (21) containing ampicillin until absorption at 600nm reached 1. Then, IPTG (Isopropyl-β-D-thiogalactopyranoside) was added to the medium followed by culturing for another 6 hours. 'After culturing, the cells were collected by centrifugation at 6,000rpm for 20min.

### Example 8 Purification of Thermostable Enzymes

The collected cells were frozen and thawed at -20°C. Next, alumina in a volume twice as that of the cells and 1mg of DNase were added to the cells, disrupting the cells. 5 volumes of 10mM Tris-hydrochloric acid buffer (pH 8.0) was added to the disrupted cells, thereby obtaining a suspension. The thus obtained suspension was heated at 85°C for 30 min, followed by centrifugation at 11,000rpm for 20 min, allowing the supernatant to adsorb to HiTrapQ column (Pharmacia). Then, elution was performed with an NaCl concentration gradient, so that active fractions were obtained. Further, the obtained active fraction solution was applied to a HiLoad 26/60 Superdex200pg gel filtration column (Pharmacia), thereby obtaining purified enzymes.

### Example 9 Measurement of Physical and Chemical Properties of Enzyme

### (1) Chemical properties of Enzymes

Determinationofprotein-codingregionsbasedonnucleotide sequence analysis and N-terminal amino acid sequence analysis revealed that this enzyme comprises 388 residues.

Further, the result of SDS polyacrylamide electrophoresis conducted on this enzyme showed that the molecular weight of this enzyme is 44,000Da.

Furthermore, gel filtration analysis using a G2000SWXL (Toso) column found that this enzyme has a homodimeric subunit structure.

Moreover, the result of isoelectric focusing of this enzyme revealed that the isoelectric point of this enzyme is 5.2.

### (2) Amino Acid Group Transfer Reaction

Enzyme reaction was conducted under conditions of pH8.0 and 25°C using 2-ketoglutaric acid as an amino group receptor and using two types of substrate as amino group donors. Then, kinetic parameters of each substrate were compared.

When an acidic substrate (aspartic acid) was used as an amino group donor, malate dehydrogenase was coupled to the reaction. Next, a change in the amount of NADH was traced with a change in absorbance at 340nm, and then kinetic parameters, Kcat and Km values were measured.

When hydrophobic substrate (phenylalanine) was used as an amino group donor, the amount of reaction product (phenylpyruvic acid) was traced with a change in absorbance at 280nm, and then Kcat and Km values were measured.

### Table 1 shows the results.

**Table 1**

| Substrate | K_{cat} (s⁻¹) | Kₘ (mM) | K_{cat}/Kₘ (s⁻¹M⁻¹) |
|---|---|---|---|
| aspartic acid 2-ketoglutaric acid | 0.18 | 105 < 0.001 | 1.7 |
| phenylalanine 2-ketoglutaric acid | 12 | 1.2 < 0.001 | 1.0 × 10⁴ |

As shown in Table 1, Kcat of this enzyme for phenylalanine and aspartic acid was 12 and 0.18sec⁻¹ (25°C, pH8.0), respectively; the Kcat/Km value for the same was 1.0x10⁴ and 1.7sec⁻¹M⁻¹, respectively. Therefore, it was shown that this enzyme is an aminotransferase having higher catalytic activity for an aromatic amino acid, e.g. phenylalanine than that for a non-aromatic amino acid, e.g. aspartic acid.

### (3) Optimum Temperature and Optimum pH

Optimum temperature and optimum pH were measured as described below using L-cysteic acid and 2-ketoglutaric acid as substrates.

Optimum temperature was determined according to the temperature dependence of the Kapp value which was determined by varying the reaction temperature from 30°C to 98°C in 50mM phosphate buffer (pH6.5), tracing an increase in absorbance at 412nm resulting from reduction of 5,'5'-Dithiobis (2-nitrobenzoic acid) (DTNB), finding Kapp value from the initial velocity.

Optimum pH was determined according to pH dependence of the Kapp value which was determined under the measurement conditions as described above by maintaining the reaction temperature at 30°C, and varying the pH of an enzyme reaction solution from 3.4 to 7.5, finding Kapp.

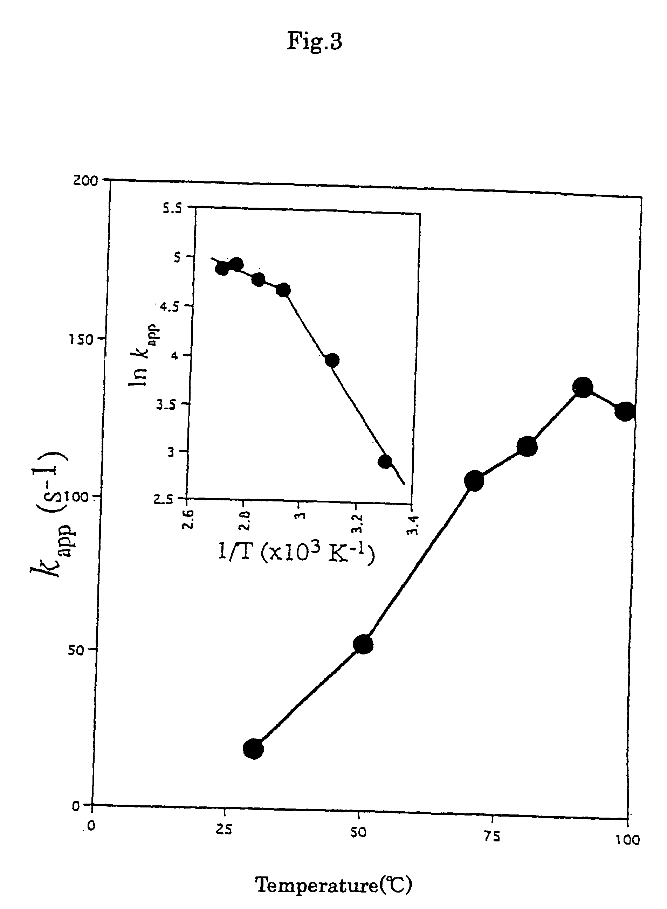

Figure 3 shows the results.

As shown in Fig. 3, when L-cysteic acid and 2-ketoglutaric acid were used as substrates, Kapp value increased as the temperature rose, and peaked at 90°C. At this time, Kapp value was 1.39x10²sec⁻¹ (pH6.5, 90°C). Thus, the optimum temperature and the optimum pH of this enzyme was found to be 90°C and 6.0, respectively.

### (4) Thermal Stability

Thermal stability was analyzed by measurement of residual activity after heating and with a differential scanning calorimeter (DSC).

To measure residual activity after heating, this enzyme (0.1mg/ml) was heated for a certain period of time at 95°C and at 110°C in 20mM phosphate buffer (pH6.5) and quenched. Then, residual activity was measured.

In measurement with DSC, a DSC (type CSC5100, Calolimetry Science) was used. Cell temperature was increased from 0 to 125°C (1K/min), and then a change in thermal capacity of the enzyme protein in 20mM phosphate buffer (pH6.5) was measured. Enzyme concentration employed was 1mg/ml.

As a result of measurement of residual activity after heating, the enzyme following treatment at pH6.5 and 95°C for 6 hours remained stable, or was not deactivated. Further, it was found that the enzyme has a half-life at 110°C of 30min.

The results of DSC measurement revealed that the melting temperature (Tm value) was 120.1°C (at pH 6.5) at which the enthalpy change is 2.4x10³KJ/mole. Moreover, its denaturation was irreversible.

### (5) pH Stability

pH stability was analyzed using a circular dichrograph (CD, type J-720W, JASCO Corporation). The pH of an enzyme solution (0.1mg/ml) was varied from 1.0 to 13.0, a change in intensity of negative ellipicity[θ] at 25°C was measured, so that pH stability was found.

The results revealed that α-helix content of this enzyme at 25°C and pH6.5 is 40%, and the enzyme remains stable over a wide pH range from 4 to 11 for 24 hours or more.

The results from (4) and (5) suggest that this enzyme shows extremely high thermal stability and pH stability.

All the documents cited in this specification are incorporated into the specification as references in their entirety.

### INDUSTRIAL APPLICABILITY

The present invention provides aminotransferase which remains stable at high temperature and over a wide pH range. The aminotransferase of this invention is useful as a catalyst for aminotransferase reaction under severe conditions. Particularly, the aminotransferase of this invention is useful as a catalyst of aminotransferase reaction using aromatic amino acid as a substrate, since the aminotransferase has very high aminotransferase activity for aromatic amino acid. Aminotransferase reaction using the aminotransferase of this invention can yield amino acid derivatives with high optical purity.

Furthermore, the present invention provides a gene encoding the aminotransferase of this invention. The gene of this invention is useful in production of the aminotransferase of this invention. That is, the protein of this invention can be produced in large quantity by integrating the gene of this invention into an expression vector, and introducing the vector into a host cell for expression.

## Claims

1. An enzyme which:
(1) has aminotransferase activity,
(2) exhibits higher aminotransferase activity when an aromatic amino acid is used as an amino group donor rather than when a non-aromatic amino acid is used as an amino group donor, and
(3) has an optimum temperature of 90°C.

2. An enzyme which:
(1) has aminotransferase activity,
(2) exhibits higher aminotransferase activity when an aromatic amino acid is used as an amino group donor than when a non-aromatic amino acid is.used as an amino group donor,
(3) has an optimum temperature of 90°C,
(4) has an optimum PH of 6.0,
(5) maintains its activity even when subjected to treatment at pH 6.5 and 95°C for 6 hours,
(6) has a half-life at pH 6.5 and 110°C of 30 minutes,
(7) remains stable at pH 4 to pH 11 and 25°C for 24 hours or more,
(8) has a melting temperature at pH 6.5 of 120.1°C where molar enthalpy change is 2.4x10³KJ/mole,
(9) has an α-helix content of 40% at pH 6.5 and 25°C,
(10) has molecular weight of 44,000Da,
(11) has a homodimeric subunit structure,
(12) has an isoelectric point of 5.2, and for which;
(13) denaturation is irreversible.

3. A protein which is the following (a) or (b):
(a) a protein which comprises an amino acid sequence of SEQ ID NO: 1,
(b) a protein which comprises an amino acid sequence derived from the amino acid sequence of SEQ ID NO: 1 by deletion, replacement or addition of one or more amino acids and has aminotransferase activity.

4. A gene which encodes the protein of claim 3.


SEQUENCE LISTING

<110> Japan as represented by Director-General of Agency of Industrial
Science and Technology

<120> A thermostable enzyme having aminotransferase activity and a gene
coding the enzyme

<130> PH-640-PCT

<160> 2

<210> 1
<211> 389
<212> PRT
<213> Pyrococcus horikoshi

<400> 1
Met Ala Leu Ser Asp Arg Leu Glu Leu Val Ser Ala Ser Glu Ile Arg
1               5                   10                  15

Lys Leu Phe Asp Ile Ala Ala Gly Met Lys Asp Val Ile Ser Leu Gly
            20                  25                  30

Ile Gly Glu Pro Asp Phe Asp Thr Pro Gln His Ile Lys Glu Tyr Ala
        35                  40                  45

Lys Glu Ala Leu Asp Lys Gly Leu Thr His Tyr Gly Pro Asn Ile Gly
    50                  55                  60
Leu Leu Glu Leu Arg Glu Ala Ile Ala Glu Lys Leu Lys Lys Gln Asn
65                  70                  75                  80
Gly Ile Glu Ala Asp Pro Lys Thr Glu Ile Met Val Leu Leu Gly Ala
                85                  90                  95
Asn Gln Ala Phe Leu Met Gly Leu Ser Ala Phe Leu Lys Asp Gly Glu
            100                 105                 110
Glu Val Leu Ile Pro Thr Pro Ala Phe Val Ser Tyr Ala Pro Ala Val
        115                 120                 125
Ile Leu Ala Gly Gly Lys Pro Val Glu Val Pro Thr Tyr Glu Glu Asp
    130                 135                 140
Glu Phe Arg Leu Asn Val Asp Glu Leu Lys Lys Tyr Val Thr Asp Lys
145                 150                 155                 160
Thr Arg Ala Leu Ile Ile Asn Ser Pro Cys Asn Pro Thr Gly Ala Val
                165                 170                 175
Leu Thr Lys Lys Asp Leu Glu Glu Ile Ala Asp Phe Val Val Glu His
            180                 185                 190
Asp Leu Ile Val Ile Ser Asp Glu Val Tyr Glu His Phe Ile Tyr Asp
        195                 200                 205

Asp Ala Arg His Tyr Ser Ile Ala Ser Leu Asp Gly Met Phe Glu Arg
    210                 215                 220

Thr Ile Thr Val Asn Gly Phe Ser Lys Thr Phe Ala Met Thr Gly Trp
225                 230                 235                 240

Arg Leu Gly Phe Val Ala Ala Pro Ser Trp Ile Ile Glu Arg Met Val
                245                 250                 255

Lys Phe Gln Met Tyr Asn Ala Thr Cys Pro Val Thr Phe Ile Gln Tyr
            260                 265                 270

Ala Ala Ala Lys Ala Leu Lys Asp Glu Arg Ser Trp Lys Ala Val Glu
        275                 280                 285

Glu Met Arg Lys Glu Tyr Asp Arg Arg Arg Lys Leu Val Trp Lys Arg
    290                 295                 300

Leu Asn Glu Met Gly Leu Pro Thr Val Lys Pro Lys Gly Ala Phe Tyr
305                 310                 315                 320

Ile Phe Pro Arg Ile Arg Asp Thr Gly Leu Thr Ser Lys Lys Phe Ser
                325                 330                 335

Glu Leu Met Leu Lys Glu Ala Arg Val Ala Val Val Pro Gly Ser Ala
            340                 345                 350

Phe Gly Lys Ala Gly Glu Gly Tyr Val Arg Ile Ser Tyr Ala Thr Ala

        355                 360                 365

Tyr Glu Lys Leu Glu Glu Ala Met Asp Arg Met Glu Arg Val Leu Lys
    370                 375                 380

Glu Arg Lys Leu Val
385 389

<210> 2
<211> 1170
<212> DNA
<213> Pyrococcus horikoshi

<400> 2
   atg gcg cta agt gac aga tta gaa tta gtt agt gct tct gaa att aga 48
   Met Ala Leu Ser Asp Arg Leu Glu Leu Val Ser Ala Ser Glu Ile Arg
   1 5 10 15
   aag ctc ttt gat att gct gca gga atg aag gat gtt atc tcc ctg gga 96
   Lys Leu Phe Asp Ile Ala Ala Gly Met Lys Asp Val Ile Ser Leu Gly
   20 25 30
   ata ggg gaa cct gat ttt gat acg cct caa cat att aag gag tat gcc 144
   Ile Gly Glu Pro Asp Phe Asp Thr Pro Gln His Ile Lys Glu Tyr Ala
   35 40 45
   aag gaa gcc ctg gat aag gga ttg act cat tat ggt cca aat ata ggg 192
   Lys Glu Ala Leu Asp Lys Gly Leu Thr His Tyr Gly Pro Asn Ile Gly
   50 55 60
   ctt tta gag ctt agg gaa gcc ata gct gaa aag tta aag aag cag aat 240
   Leu Leu Glu Leu Arg Glu Ala Ile Ala Glu Lys Leu Lys Lys Gln Asn
   65 70 75 80
   ggc ata gag gct gat cca aag aca gag ata atg gtc tta tta ggt gcg 288
   Gly Ile Glu Ala Asp Pro Lys Thr Glu Ile Met Val Leu Leu Gly Ala
   85 90 95
   aac caa gct ttc tta atg ggc ctc tcg gct ttt ctt aag gat ggt gaa 336
   Asn Gln Ala Phe Leu Met Gly Leu Ser Ala Phe Leu Lys Asp Gly Glu
   100 105 110
   gag gta tta ata cca act cca gcc ttt gtc agc tac gca cct gcc gtt 384
   Glu Val Leu Ile Pro Thr Pro Ala Phe Val Ser Tyr Ala Pro Ala Val
   115 120 125
   ata ttg gct gga gga aag ccc gtt gaa gtc cca acg tac gaa gag gat 432
   Ile Leu Ala Gly Gly Lys Pro Val Glu Val Pro Thr Tyr Glu Glu Asp
   130 135 140
   gaa ttc agg cta aac gtt gat gag ctt aaa aag tat gtt acc gac aag 480
   Glu Phe Arg Leu Asn Val Asp Glu Leu Lys Lys Tyr Val Thr Asp Lys
   145 150 155 160
   act aga gct tta ata ata aac tca ccg tgt aat cca acg gga gca gtg 528
   Thr Arg Ala Leu Ile Ile Asn Ser Pro Cys Asn Pro Thr Gly Ala Val
   165 170 175
   tta act aag aaa gat cta gaa gag ata gcg gat ttt gtc gtt gaa cat 576
   Leu Thr Lys Lys Asp Leu Glu Glu Ile Ala Asp Phe Val Val Glu His
   180 185 190
   gat cta att gta ata agc gat gaa gtt tat gag cac ttc att tac gat 624
   Asp Leu Ile Val Ile Ser Asp Glu Val Tyr Glu His Phe Ile Tyr Asp
   195 200 205
   gat gct agg cac tac agt ata gcc tcc ctg gat gga atg ttt gaa agg 672
   Asp Ala Arg His Tyr Ser Ile Ala Ser Leu Asp Gly Met Phe Glu Arg
   210 215 220
   aca ata acc gtt aac gga ttc tca aag acg ttt gca atg acg ggc tgg 720
   Thr Ile Thr Val Asn Gly Phe Ser Lys Thr Phe Ala Met Thr Gly Trp
   225 230 235 240
   agg ttg gga ttt gtt gca gcg cct tct tgg ata ata gag agg atg gtg 768
   Arg Leu Gly Phe Val Ala Ala Pro Ser Trp Ile Ile Glu Arg Met Val
   245 250 255
   aag ttt cag atg tat aac gct act tgt cca gtg act ttc ata caa tac 816
   Lys Phe Gln Met Tyr Asn Ala Thr Cys Pro Val Thr Phe Ile Gln Tyr
   260 265 270
   gct gct gct aaa gcg tta aag gat gag aga agc tgg aaa gct gtt gaa 864
   Ala Ala Ala Lys Ala Leu Lys Asp Glu Arg Ser Trp Lys Ala Val Glu
   275 280 285
   gag atg aga aag gag tac gac aga aga aga aag ctc gtg tgg aag agg 912
   Glu Met Arg Lys Glu Tyr Asp Arg Arg Arg Lys Leu Val Trp Lys Arg
   290 295 300
   ctt aac gag atg gga ctc cca acg gta aag ccg aag ggt gca ttt tac 960
   Leu Asn Glu Met Gly Leu Pro Thr Val Lys Pro Lys Gly Ala Phe Tyr
   305 310 315 320
   ata ttc ccg agg ata agg gat act ggg cta acg agc aag aaa ttc agc 1008
   Ile Phe Pro Arg Ile Arg Asp Thr Gly Leu Thr Ser Lys Lys Phe Ser
   325 330 335
   gag ctc atg ctt aaa gaa gct agg gtt gca gta gtt cca ggt agt gcc 1056
   Glu Leu Met Leu Lys Glu Ala Arg Val Ala Val Val Pro Gly Ser Ala
   340 345 350
   ttt gga aaa gcc ggt gag gga tac gta agg atc agc tat gca aca gct 1104
   Phe Gly Lys Ala Gly Glu Gly Tyr Val Arg Ile Ser Tyr Ala Thr Ala
   355 360 365
   tat gag aag ctt gaa gag gcc atg gat aga atg gaa agg gtg tta aag 1152
   Tyr Glu Lys Leu Glu Glu Ala Met Asp Arg Met Glu Arg Val Leu Lys
   370 375 380
   gag agg aag cta gtt taa 1170
   Glu Arg Lys Leu Val
   385 389